(19) Europäisches Patentamt
European Patent Office
Office européen des brevets

(11) Publication number: **0 432 945 A1**

# (12) EUROPEAN PATENT APPLICATION

(21) Application number: **90313044.1**

(51) Int. Cl.[5]: **A61L 15/44**, A61L 15/58

(22) Date of filing: **30.11.90**

(30) Priority: **12.12.89 US 449037**

(43) Date of publication of application:
**19.06.91 Bulletin 91/25**

(84) Designated Contracting States:
**AT BE CH DE DK ES FR GB GR IT LI LU NL SE**

(71) Applicant: **WARNER-LAMBERT COMPANY**
**201 Tabor Road**
**Morris Plains New Jersey 07950(US)**

(72) Inventor: **Granger, Colin D.**
**615 Hillside Road**
**Chester, New Jersey 07930(US)**

(74) Representative: **Jones, Michael Raymond et al**
**HASELTINE LAKE & CO. Hazlitt House 28**
**Southampton Buildings Chancery Lane**
**London WC2A 1AT(GB)**

## (54) A transdermal delivery system for treatment of cocaine and heroin addiction.

(57) For the treatment of cocaine and heroin addiction in a human or animal subject, there is disclosed a transdermal delivery system which includes as the active ingredient buprenorphine or a pharmaceutically-acceptable salt.

EP 0 432 945 A1

## A TRANSDERMAL DELIVERY SYSTEM FOR TREATMENT OF COCAINE AND HEROIN ADDICTION

Buprenorphine has been shown, in both animal and human studies, to be effective in decreasing the daily requirements of morphine and heroin in opiate-addicted subjects (Mello, N.K. et al., "Buprenorphine Suppresses Heroin Use by Heroin Addicts", Science, Feb. 1980, Vol. 207, pp. 657-659). More recently, Mello has reported (Mello, N.K. et al., "Buprenorphine Suppresses cocaine Self-administration In Rhesus Monkeys", Science, August 1989, Vol. 245, pp. 859-862.) that intravenous buprenorphine is effective in reducing the total quantity and frequency of self-administered doses of cocaine in cocaine-addicted Rhesus monkeys.

Several patents generally describe buprenorphine as a narcotic antagonist and treatment for opiate addicts. U.S. Patent No. 4,722,928 describes N-oxide derivaties of 3-hydroxy morphinans and partial morphinans which are useful as analgesics or narcotic antagonists via oral administration. U.S. Patent No. 4,489,079 describes pharmaceutical compositions having a peripheral antagonistic action with respect to opiates containing a haloallylate of levallorphan and, optionally, narcotic analgesics such as buprenorphine. U.S. Patent No. 4,464,378 describes nasal dosage forms of narcotic antagonists and analgesics, such as buprenorphine. U.S. Patent No. 4,582,835 describes analgesic compositions comprising buprenorphine and naloxone for treatment of opiate dependent subjects.

It is also known that certain pharmaceuticals are absorbed to a degree through the skin. Commercially, the transdermal pharmaceutical absorption technique is used with nitroglycerin, scopolamine, clonidine and estradiol. However, various patents describe transdermal delivery systems, such as U.S. Patent No. 4,849,224 which describes a device for administering an active agent to the skin comprising a drug-containing reservoir defined by a backing layer, a permeable membrane layer, a ring-shaped adhesive layer and a peelable liner layer. The device is described as useful for a variety of drugs, such as anti-inflammatory drugs, analgesics, narcotic antagonists, antibiotics, tranquilizers, antihistamines, hormonal agents and many others.

U.S. Patent No. 4,844,903 describes a process for making an adhesive plaster for the transdermal administration of pharmaceutical, in particular heat-sensitive active substances, composed of an adhesive layer, a backing layer, a matrix-layer containing the active substances, and a protective layer.

U.S. Patent No. 4,557,934 describes penetrating topical pharmaceutical compositions containing a pharmaceutically-active agent and the penetration enhancing agent 1-dodecyl-azacycloheptan-2-one.

U.S. Patent No. 4,806,341 describes a transdermal absorption dosage unit for morphinan narcotic analgesics and antagonists comprising an impervious backing layer, a polymer matrix disc layer containing morphinan pharmaceutical, and adhesive means which adheres the dosage unit in contact with the skin.

U.S. Patent No. 4,626,539 describes transdermal pharmaceutical compositions consisting essentially of a therapeutically effective amount of an opioid or salt thereof, a fatty alcohol or fatty acid penetration enhancer and a suitable pharmaceutical carrier. The compositions are useful as an analgesic for the treatment of pain, as a narcotic antagonist, for the treatment of obesity or shock, sexual dysfunction, cognitive disorders and for treatment of stroke.

According to the present invention, there is provided a transdermal delivery system intended for the treatment of cocaine or heroin addiction, the system including, as the active ingredient to be administered transdermally, buprenorphine or a pharmaceutically acceptable salt thereof.

The delivery system of the present invention provides means for the treatment of cocaine and heroin addiction, which provides the following advantages:

1. a therapeutic effect which is fairly constant;
2. smoothness and consistency of the levels of buprenorphine;
3. reduced potential of buprenorphine abuse or addition;
4. decreased risk of overdosing and resulting toxic reactions;
5. improvements in the ability of heroin and cocaine addicts correctly and safely to use buprenorphine as a treatment on an outpatient, unsupervised basis; and
6. improved patient compliance with a recommended treatment program.

The transdermal delivery system can comprise a transdermal adhesive patch which contains from 0.25 milligrams to 100 milligrams of buprenorphine or salt thereof. The transdermal delivery system can also take the form of a cream or ointment of which from 0.1 to 20% by weight is constituted by buprenorphine or the salt thereof.

The exact mechanism by which buprenorphine exerts this effect is not known, although applicant believes (but not intending to be limited thereby) that the effectiveness of buprenorphine in this regard is a result of its potent agonistic effect upon the u centers of the central nervous system.

Although buprenorphine is absorbed following oral and sub-lingual administration, these routes are not advantageous for self, unsupervised administration of buprenorphine to cocaine and heroin addicts. As the half-life of the drug in the body after administration is approximately three hours, the oral or sub-lingual routes would require frequent administration in order to provide effective and continuous levels of buprenorphine in the blood. However, transdermal delivery according to the present invention would provide constant and consistent blood levels of buprenorphine which are effective to reduce or eliminate the physiological symptoms of cocaine and heroin addiction.

Buprenorphine is a highly lipophilic ring-C-bridged oripavine of thebaine which has the chemical structure below:

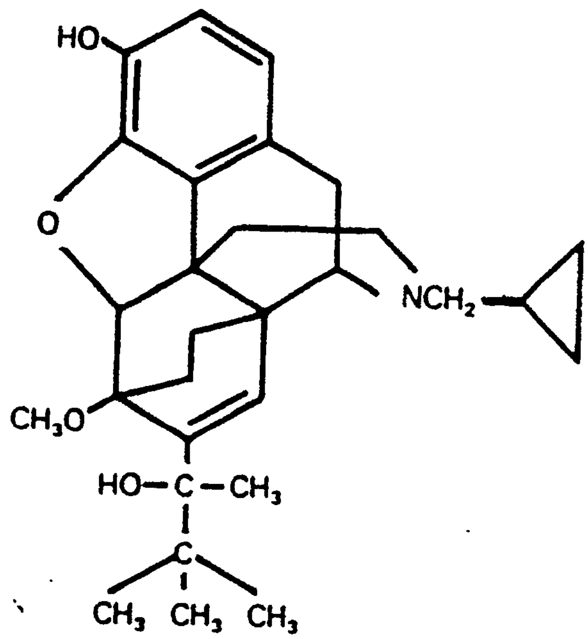

The pharmaceutically-acceptable salts of buprenorphine useful in this invention include any of the non-toxic salts of buprenorphine which have pharmacologic properties, such as, for example, the hydrochloride, hydrobromide, hydroiodide, sulfate, bisulfate, nitrate, citrate, tartrate, bitartrate, lactate, phosphate, malate, maleate, fumarate, succinate, acetate, pameate, stearate, oleate, palmitate, laurate, valerate and the like. The preferred salt is the hydrochloride.

A "therapeutically-effective" amount of buprenorphine or salt thereof in the blood is the blood levels equivalent to those obtained following the administration of from about 0.1 milligram to about 0.6 milligram of buprenorphine by the intravenous route. Typical "therapeutically-effective" levels of buprenorphine in the blood ranges from about 0.1-100 nanograms per milliliter of blood plasma; preferably from about 0.1 to about 30 nanograms/millilter; more preferably from about 0.6 to about 6 nanograms/milliliter. Transdermal delivery of the buprenorphine or salt according to the present invention can be by a variety of topical applications to the external skin of the body, such as, for example, by an adhesive patch, film, spray, cream or ointment and the like. It is preferred that transdermal delivery of buprenorphine or salt be by topical application of an adhesive patch, cream or ointment.

The transdermal adhesive patch useful in this invention can be any of the transdermal patches known in the art and generally includes a laminated composite device that includes a reservoir layer containing the buprenorphine, a pressure-sensitive adhesive layer for attaching the device to the skin, and an impermeable backing layer. Some examples of suitable adhesive patch devices are described in U.S. Patent Nos. 4,849,224; 4,806,341, and 4,626,539, the disclosures of which are herein incorporated by reference and made a part hereof. Preferably the adhesive patch of this invention comprises an inert, impermeable film backing containing an acrylic adhesive which has incorporated therein buprenorphine or salt thereof. By the term "acrylic adhesive" is meant adhesives prepared from polymers and copolymers of acrylic esters and copolymers of acrylic esters and other ethylenically-unsaturated monomers. Preferably the acrylic adhesive is selected from the group consisting of butyl acrylate, ethyl acrylate, ethyl hexyl acrylate,

vinylacetate/ethylene acrylate and mixtures thereof. The acrylic adhesives of this invention can be prepared by conventional processes which are well known in the art, including suspension, dispersion, emulsion or solution polymerization techniques.

The adhesive patch useful in this invention preferably comprises an effective amount of buprenorphine or salt thereof, to deliver and maintain blood levels equivalent to those obtained following the intravenous administration of from about 0.1 milligram to about 0.6 milligram of buprenorphine and defined earlier as "therapeutically-effective" levels. Preferably, the adhesive patch comprises from about 0.25 milligram to about 100 milligrams of buprenorphine or salt thereof. The adhesive patch may also include one or more skin permeation enhancers such as saturated or unsaturated fatty acids having 8-18 carbon atoms or $C_1$ - $C_4$ alkyl ester thereof, monoglycerides, acetates, diethanolamides and N,N-dimethylamides, such as oleic acid, propyl oleate, oleyl acetate, propyl myristate, myristyl alcohol, stearic acid, stearyl alcohol, myristyl N,N-dimethyl amide, stearyl propyl ester, capryl alcohol, ethyl caprylate, hexamethylene lauramide, hexamethylene palmitate and the like.

The transdermal adhesive patch of this invention can also contain an acceptable vehicle, such as for example propylene glycol, isopropanol, ethanol, n-methylpyrolidone and the like, as well as other optional ingredients such as thickeners, emulsifiers, stabilizers, pharmaceuticals and other conventional additives used in topical pharmaceutical compositions.

The impermeable film backing used in the adhesive patch of the present invention can be any inert, flexible material such as polyethylene, polyester, polypropylene, polyvinyl chloride, polystyrene, polyethylene terephthalate, or combinations thereof, and may contain one more metal layers and/or one or more layers of natural or synthetic fibers. This film backing may be composed of a single film or a plurality of films.

The method of this invention can also be carried out utilizing a cream, ointment or spray which comprises a sufficient amount of buprenorphine or salt thereof to transdermally deliver and maintain "therapeutically-effective levels of buprenorphine in the bloodstream. Preferably the cream, ointment or spray comprises from about 0.1 to about 20% by weight of buprenorphine or salt thereof. The cream, ointment or spray may also contain additional ingredients such as skin permeation enhancers, vehicles, thickeners, stabilizers, pharmaceuticals, emulsifiers and other additives as described earlier.

The method of the present invention is practiced by topically applying the transdermal adhesive patch, cream, ointment or spray to the external skin of the cocaine or heroin-addicted subject. The application is repeated as needed depending on the amount of buprenorphine or salt thereof in the patch, cream, ointment or spray.

The following examples further illustrate this invention but are not intended to limit the invention in any manner.

Example I

A cream or ointment composition accoridng to the present invention can be prepared according to the formulation shown in Table I. The composition can then be topically applied to the external skin of a cocaine- or heroin-addicted subject, typically at a surface area of about 1 square inch to about 10 square inches. The composition is effective in transdermally delivering and maintaining about 0.1 to about 100 nanograms of buprenorphine per milliliter of blood plasma or greater in the bloodstream of the addicted subject, and is effective in reducing or eliminating the physiological dependency of the addicted subject on cocaine or heroin.

Table I

| Ingredient | Amount (weight %) |
|---|---|
| buprenorphine or salt thereof | 0.1 - 20% |
| vehicle | 20 - 90% |
| skin permeation enchancer | 1 - 30% |
| other additives | 0 - 80% |

Example II

A transdermal adhesive patch according to this invention can be prepared by homogenously mixing from about 0.25 milligram to about 100 milligrams of buprenorphine or salt thereof in a vehicle with an acrylic emulsion. This mixture can then be spread upon an impermeable polymeric film backing and dried until the adhesivie is firm, yet tacky. The coated film can be cut into any desired shape from about 1-10 square inches and topically applied and adhered to the external skin of a cocaine- or heroin- addicted subject. The patch is effective in transdermally delivering and maintaining from about 0.1 to about 100 nanograms of buprenorphine per milliliter of blood plasma or greater in the bloodstream of the addicted subject and is effective in reducing or eliminating the physiological dependency of the addicted subject on cocaine or heroin.

## Claims

1. A transdermal delivery system intended for the treatment of cocaine or heroin addiction, the system including, as the active ingredient to be administered transdermally, buprenorphine or a pharmaceutically acceptable salt thereof.

2. A transdermal delivery system according to claim 1, which includes an adhesive patch.

3. A transdermal delivery system according to claim 2, wherein the patch contains from 0.25 milligram to 100 milligrams of buprenorphine or the salt thereof.

4. A transdermal delivery system according to claim 2 or 3, wherein the adhesive patch comprises an inert, impermeable film backing provided with an acrylic adhesive and the buprenorphine or salt thereof.

5. A transdermal delivery system according to claim 4, wherein the acrylic adhesive is a polymer or copolymer based on butyl acrylate, ethyl acrylate, ethyl hexyl acrylate, vinyl acetate/ethylene acrylate, or mixtures thereof.

6. A transdermal delivery system according to claim 1, which is in the form of a cream or ointment.

7. A transdermal delivery system according to claim 6, wherein the buprenorphine or salt thereof constitutes from 0.1 to 20% by weight of the cream or ointment.

8. A transdermal delivery system according to any preceding claim, capable of causing the introduction into the bloodstream of the subject of from 0.1 to 100 nanograms of buprenorphine or salt thereof per millilitre of blood plasma.

9. A transdermal delivery system according to claim 8, capable of causing the introduction into the bloodstream of the subject of from 0.1 to 30 nanograms per millilitre of blood plasma.

10. A transdermal delivery system according to claim 8, capable of causing the introduction into the bloodstream of the subject of from 0.6 to 6 nanograms per millilitre of blood plasma.

Claims for the following Contracting States: ES, GR

1. A process for producing a transdermal delivery system intended for the treatment of cocaine or heroin addiction, which comprises incorporating into the system, as the active ingredient to be administered transdermally, buprenorphine or a pharmaceutically acceptable salt thereof.

2. A process for producing a transdermal delivery system according to claim 1, which includes incorporating the active ingrediant in an adhesive patch.

3. A process for producing a transdermal delivery system according to claim 2, which includes incorporating from 0.25 milligram to 100 milligrams of buprenorphine or the salt thereof in the patch.

4. A process for producing a transdermal delivery system according to claim 2 or 3, which includes incorporating in the adhesive patch an inert, impermeable film backing provided with an acrylic adhesive and the buprenorphine or salt thereof.

5. A process for producing a transdermal delivery system according to claim 4, wherein the acrylic adhesive is a polymer or copolymer based on butyl acrylate, ethyl acrylate, ethyl hexyl acrylate, vinyl acetate/ethylene acrylate, or mixtures thereof.

6. A process for producing a transdermal delivery system according to claim 1, which includes incorporating the active ingredient in a cream or ointment.

7. A process for producing a transdermal delivery system according to claim 6, wherein the buprenorphine or salt thereof constitutes from 0.1 to 20% by weight of the cream or ointment.

8. A process for producing a transdermal delivery system according to any preceding claim, capable of causing the introduction into the bloodstream of the subject of from 0.1 to 100 nanograms of buprenorphine or salt thereof per millilitre of blood plasma.

9. A process for producing a transdermal delivery system according to claim 8, capable of causing the introduction into the bloodstream of the subject of from 0.1 to 30 nanograms per millilitre of blood plasma.

10. A process for producing a transdermal delivery system according to claim 8, capable of causing the introduction into the bloodstream of the subject of from 0.6 to 6 nanograms per millilitre of blood plasma.

European Patent Office

# EUROPEAN SEARCH REPORT

Application Number

EP 90 31 3044

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | US-A-4 844 903 (PAWAN SETH)<br>* claims 1, 2 * | 1-3 | A 61 L 15/44<br>A 61 L 15/58 |
| D,X,D,Y | EP-A-0 171 742 (E.I.DU PONT DE NEMOURS AND COMPANY)<br>* page 7, lines 19 - 26 * * page 8, lines 30 - 35 * * page 10, lines 6 - 35 * * page 11, lines 1 & US-A-4626539 * | 1,2-10 | |
| X,Y | WO-A-8 809 676 (WARNER-LAMBERT COMPANY)<br>* page 4, lines 12 - 20 * * page 5, lines 3 - 18 * | 1,2-10 | |
| D,A | EP-A-0 316 168 (THERATECH, INC)<br>* column 4, lines 50 - 52 * * column 5, lines 9 - 10 & US-A-4849224 * | 1-10 | |
| D,A | SCIENCE. vol. 245, 25 August 1989, LANCASTER, PA US pages 859 - 862; Nancy K. Mello et al.: "Buprenorphine suppresses Cocaine self-administration by Rhesus Monkeys"<br>* abstract * | 1 | |

TECHNICAL FIELDS SEARCHED (Int. Cl.5)

A 61 L

The present search report has been drawn up for all claims

| Place of search | Date of completion of search | Examiner |
|---|---|---|
| The Hague | 22 March 91 | ESPINOSA Y CARRETERO |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document